(19) Europäisches Patentamt
European Patent Office
Office européen des brevets

(11) Veröffentlichungsnummer: **0 296 366 B1**

(12) **EUROPÄISCHE PATENTSCHRIFT**

(45) Veröffentlichungstag der Patentschrift: **18.09.91**

(51) Int. Cl.$^5$: **G01N 33/549**, //G01N33/78, G01N33/94,G01N33/558

(21) Anmeldenummer: **88108187.1**

(22) Anmeldetag: **21.05.88**

(54) **Heterogenes Immunoassay.**

(30) Priorität: **29.05.87 DE 3718140**

(43) Veröffentlichungstag der Anmeldung:
**28.12.88 Patentblatt 88/52**

(45) Bekanntmachung des Hinweises auf die Patenterteilung:
**18.09.91 Patentblatt 91/38**

(84) Benannte Vertragsstaaten:
**AT BE CH DE ES FR GB GR IT LI LU NL SE**

(56) Entgegenhaltungen:
**EP-A- 0 167 171**
**DE-A- 2 934 110**
**US-A- 4 385 126**

(73) Patentinhaber: **BOEHRINGER MANNHEIM GMBH**
**Sandhofer Strasse 116**
**W-6800 Mannheim 31(DE)**

(72) Erfinder: **Mangold, Dieter, Dr.**
**Fröhlichstr. 62**
**W-6800 Mannheim 1(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein heterogenes immunologisches Verfahren zur Bestimmung eines Analyten in einer Losung, bei dem mindestens ein Partner einer immunologischen Reaktion vor oder/und während der Inkubation von einem Trägermaterial gelöst wird.

Zu den heterogenen Immunoassays zählen beispielsweise kompetitive Assays. Solche Testverfahren beinhalten im allgemeinen den Wettstreit zwischen einer bekannten Menge markiertem und einer unbekannten Menge unmarkiertem, zu bestimmendem Analyten um eine begrenzte Menge eines analytspezifischen festphasengebundenen Partners einer immunologischen Reaktion. Nach Durchführung der immunologischen Reaktion kann der festphasengebundene markierte Analyt von dem freien markierten Analyten getrennt und mittels der Markierung seine Menge chemisch oder physikalisch bestimmt werden. Die Größe des ermittelten Meßwertes ist umgekehrt proportional zur Menge des zu bestimmenden unmarkierten Analyten. Als Alternative kann auch die Menge des ungebunden gebliebenen markierten Analyten bestimmt werden. In diesem Falle ist die Größe des Meßwertes direkt proportional zur Menge des zu bestimmenden Analyten.

Ein Beispiel für solche kompetitiven heterogenen Immunoassays sind ELISA-Teste (enzyme linked immunosorbent assay), bei denen als Analyt in einer Probe üblicherweise Antigene bestimmt werden, die mit markierten Antigenen um festphasengebundene Antikörper konkurrieren. Als Markierung wird ein Enzym verwendet, wie z. B. β-D-Galactosidase, Peroxidase oder alkalische Phosphatase. Prinzipiell können in kompetitiven Immunoassays jedoch alle möglichen Markierungen, wie z. B. radioaktive Isotope, Fluoreszenzmarker etc. eingesetzt werden.

Ein anderer heterogener Immunoassay, bei dem ein Enzym als Markierung verwendet wird, ist der "immunoenzymometric assay" (IEMA). Im Gegensatz zu den ELISA-Testen werden hier beispielsweise zur Bestimmung von Antigenen markierte Antikörper und festphasengebundene Antigene eingesetzt. Zuerst findet in homogener Phase die Reaktion zwischen Antigen und einem Überschuß an markiertem Antikörper statt. Dann wird der Überschuß an markiertem Antikörper durch festphasengebundenes Antigen entfernt und anschließend die Menge der festphasengebundenen Markierung oder der ungebundenen Markierung bestimmt. Auch hier ist im Falle der Bestimmung der festphasengebundenen Markierung die Größe des Meßwertes umgekehrt proportional zur Menge des zu bestimmenden unmarkierten Antigens in der Probe. Die Größe des Meßwertes für die nicht gebundene Markierung ist direkt proportional zur Menge des zu bestimmenden Antigens.

Sehr oft werden heterogene Immunoassays so ausgeführt, daß die einzelnen Reaktionspartner und Reagenzien manuell zusammengegeben werden und anschließend inkubiert, die feste von der flüssigen Phase manuell getrennt und gegebenenfalls nach einem Waschschritt die Menge der Markierung bestimmt wird. Um solche Immunoassays zu vereinfachen, ihre Empfindlichkeit und Reproduzierbarkeit zu erhöhen, Zeit und Personalkosten zu sparen, wurden trägergebundene Tests oder automatisierte Testführungen entwickelt.

Beispielsweise ist im USP 4,446,232 eine Vorrichtung zur Bestimmung von Antigenen beschrieben, welche aus zwei Zonen besteht. In der ersten Zone befinden sich enzymmarkierte Antikörper und immobilisierte Antigene. Die zweite Zone enthält Material, welches in der Lage ist, mit den enzymmarkierten Antikörpern zu reagieren und eine Farbe zu produzieren. Wird eine Antigene enthaltende Probe auf eine solche Vorrichtung aufgegeben, reagieren die zu bestimmenden Antigene mit den enzymmarkierten Antikörpern. Überschüssige Antikörper werden über die immobilisierten Antigene an den Träger gebunden. Antikörper, die mit zu bestimmendem freiem Antigen reagiert haben, werden mit der Flüssigkeit der Probe in die zweite Zone transportiert und verursachen dort aufgrund ihrer Enzymmarkierung die Bildung einer Farbe, deren Intensität der zu bestimmenden Antigenkonzentration in der Probe proportional ist.

EP-A 0 167 171 befaßt sich mit einem Verfahren und einer Vorrichtung zur automatisierten Durchführung analytischer, wie z. B. immunologischer Bestimmungen. Zur Bestimmung eines Haptens oder Antigens in einer Probeflüssigkeit wird ein Antikörper-Enzymkonjugat, das sich in bekannter Menge auf einem Reagenzträger befindet, von diesem eluiert und mit der Probelösung inkubiert. Es bildet sich ein löslicher Komplex aus Hapten bzw. Antigen und Konjugat, der neben überschüssigem freiem Konjugat vorliegt. Unter dem Einfluß von Kraftänderungszyklen wird diese Mischung dann einem Bereich zugeführt, in dem weiteres Hapten bzw. Antigen in unlöslicher Phase vorliegt. Noch vorhandenes überschüssiges Konjugat wird dort festgehalten, während die den Haptenkonjugatkomplex bzw. Antigenkonjugatkomplex enthaltende Flüssigkeit zu einem Trockenreagenz zur Bestimmung des im Konjugat gebundenen Enzyms weitertransportiert wird, dieses auflöst bzw. eluiert und in einem weiteren Kraftänderungszyklus schließlich zu einer Meßküvette transportiert wird, in der eine durch das Enzymreagenz hervorgerufene physikalische Änderung gemessen wird.

Trägergebundenen Tests und automatisierten Testführungen ist oft gemeinsam, daß ein markierter Reaktionspartner einer immunologischen Reaktion in bekannter Konzentration und Menge in einem Trägermaterial vorliegt und aus dieser so eluiert werden muß, daß auch die eluierte Menge des markierten Reaktionspartners genau bekannt ist. Zur Bestimmung der Markierung als letzter Schritt heterogener Immunoassays wird der Meßwert mittels einer Eichkurve der Analyt-Konzentration zugeordnet, die den zu bestimmenden Wert darstellt. Um reproduzierbare Ergebnisse zu erzielen, müssen die zu bestimmenden Proben unter den gleichen Bedingungen gemessen werden wie die zur Erstellung der Eichkurve verwendeten Proben bekannter Konzentration. Wichtig für die Reproduzierbarkeit und Präzision von trägergebundenen oder automatisierten heterogenen Immunoassays ist vor allem die Kenntnis der genauen Konzentration des markierten Reaktionspartners der immunologischen Reaktion in der Inkubationslösung. Probleme können sich dann ergeben, wenn unterschiedliche Chargen an Trägermaterialien, markierten immunologischen Reaktionspartnern oder mit diesen imprägnierten Trägermaterialien vorliegen.

Die eluierte Konzentration des markierten Reaktionspartners einer immunologischen Reaktion, z. B. des Enzym-Antikörper-Konjugats in einem IEMA-Test, ist bei trägergebundenen Tests und solchen automatisierten Testführungen, bei welchen ein mit einem Enzym-Antikörper-Konjugat imprägniertes Trägermaterial eingesetzt wird, außer von der eigentlichen Konzentration der Reaktionspartner auf dem Trägermaterial sowohl von der Qualität des Trägermaterials als auch von der Konjugatqualität abhängig. Gründe für unterschiedliche Konzentrationseinstellungen des markierten Reaktionspartners einer immunologischen Reaktion, wie in einem Test nach USP 4,446,232 oder EP-A 0 167 171, können in unterschiedlichen Chargen z. B. sein:

- Die chargenabhängige Beschaffenheit des Konjugats aus Markierung und Partner einer immunologischen Reaktion,
- unterschiedliches Saugverhalten und unterschiedliche Eluierbarkeit infolge von Inhomogenitäten des Trägermaterials
- oder unterschiedliche Konjugatmenge auf dem einzelnen Testbezirk durch inhomogene Beladung und Abmessungstoleranzen bei der Herstellung der einzelnen Testbezirke aus dem Trägermaterial.

Die hierdurch verursachten unterschiedlichen Konzentrationen an markiertem Reaktionspartner einer immunologischen Reaktion im Eluat können zu Ergebnissen mit relativ großen Variationskoeffizienten führen.

Es bestand deshalb Bedarf an einem heterogenen immunologischen Verfahren, bei dem das Ergebnis nicht durch die von Probe zu Probe möglicherweise schwankenden Mengen des von einem festen Trägermaterial eluierten markierten Reaktionspartners einer immunologischen Reaktion beeinflußt wird.

Aufgabe der vorliegenden Erfindung war es, diesen Bedarf zu befriedigen.

Gelöst wird diese Aufgabe erfindungsgemäß durch ein heterogenes immunologisches Verfahren zur Bestimmung eines Analyten in einer Probe, bei dem mindestens ein Partner der immunologischen Reaktion von einem Trägermaterial gelöst wird, wobei die analytenthaltende Probe
mit einem von einem Trägermaterial gelösten, markierten analytspezifischen Partner einer immunologischen Reaktion inkubiert,
mit einem Überschuß des zu bestimmenden Analyten in trägergebundener Form kontaktiert,
der gebundene und der freie Anteil der so entstandenen Immunkomplexe getrennt und
die Markierung im freien oder gebundenen Anteil gemessen wird,
dadurch gekennzeichnet, daß
das Trägermaterial zusätzlich eine detektierbare, die immunologische Reaktion nicht beeinflussende Substanz enthält, die zusammen mit dem Partner der immunologischen Reaktion eluiert wird
und man einen für die Konzentration der detektierbaren Substanz in der Lösung erhaltenen charakteristischen Meßwert mit dem Meßwert für die Analyt-Konzentration in der Probe in Beziehung setzt.

Hierbei können Analyte sowohl jede Art von Haptenen oder Antigenen als auch Antikörper sein.

Partner einer immunologischen Reaktion sind jeweils ein Hapten oder Antigen und ein gegen dieses Hapten oder Antigen gerichteter und mit diesem reagierender Antikörper. Solche reaktionsfähigen Partner einer immunologischen Reaktion reagieren miteinander sehr spezifisch unter Bildung von Immunkomplexen (Hapten-Antikörper- oder Antigen-Antikörper-Komplexen). Der immunologische Partner, der mit dem zu bestimmenden Analyten so reagieren kann, wird deshalb als "analytspezifisch" bezeichnet.

Als detektierbare Substanz kommen alle diejenigen Stoffe in Frage, die aufgrund ihrer physikalischen Eigenschaften, wie Radioaktivität, Fluoreszenz, Farbe usw., eine Messung ihrer Konzentration erlauben und die gleichzeitig ein vergleichbares Elutionsverhalten wie das zu eluierende Konjugat aus immunologischem Reaktionspartner und Markierung aufweisen. Als besonders geeignet haben sich fluoreszierende Verbindungen und Farbstoffe erwiesen.

Je nach der Natur des Konjugats aus immunologischem Reaktionspartner und Markierung ist die

detektierbare Kontrollsubstanz zu wählen. Diese darf weder die chemischen und physikalischen Eigenschaften des immunologischen Reaktionspartners oder der Markierung noch die Reaktion des Konjugats aus Markierung und immunologischem Reaktionspartner mit einem zu bestimmenden zweiten immunologischen Reaktionspartner beeinflussen. Auch die Messung der Markierung darf durch die detektierbare Substanz nicht gestört werden. Besonders wichtig ist es, daß die detektierbare Substanz, die zusammen mit dem Konjugat aus Markierung und immunologischem Reaktionspartner im Trägermaterial imprägniert vorliegt, die Stabilität der Markierung nicht beeinflußt. Außerdem ist es weiterhin wichtig, daß die Homogenität der Tränklösung durch die Kontrollsubstanz nicht beeinflußt wird.

Insbesondere hängt die Wahl einer detektierbaren Kontrollsubstanz von der Natur der Markierung des immunologischen Reaktionspartners ab. Wird beispielsweise ein Enzym zur Markierung des immunologischen Reaktionspartners eingesetzt, das mit Hilfe eines chromogenen Substrates nachgewiesen wird, so eignet sich als detektierbare Substanz insbesondere ein Farbstoff, dessen Absorptionsbereich hinreichend deutlich abweicht von dem Absorptionsbereich des chromogenen Substrates bzw. der Substanz, die durch die enzymatische Umsetzung hieraus entsteht. Es kann in einem solchen Fall auch eine Substanz ausgewählt werden, die mit Hilfe eines verschiedenen physikalischen Meßverfahrens detektiert werden kann, z. B. ein fluoreszierender Farbstoff oder eine radioaktiv markierte Substanz, die fluorometrisch bzw. radiometrisch gemessen werden kann. Zahlreiche weitere geeignete Kombinationen von Markierung und detektierbarer Substanz lassen sich ohne weiteres aufgrund der obigen Ausführungen ableiten. Für den speziellen Fall, daß β-Galactosidase als Markierung eingesetzt wird, die durch o-Nitrophenylgalactosid oder Chlorphenolrotgalactosid nachgewiesen werden kann, ist als detektierbare Substanzen die Gruppe der Patentblaufarbstoffe besonders geeignet. Die Absorption von o-Nitrophenol und Chlorphenolrot, die aus den entsprechenden Galactosiden durch die Galactosidase freigesetzt werden, liegt in dem Bereich unterhalb 600 nm. Die Patentblaufarbstoffe zeigen Absorptionen, die oberhalb dieses Bereiches liegen. Diese Farbstoffe können somit bei 650 nm unabhängig und ohne Störung der β-Galactosidase-Messung gemessen werden.

Patentblaufarbstoffe sind bekannte Farbstoffe mit folgender Struktur:

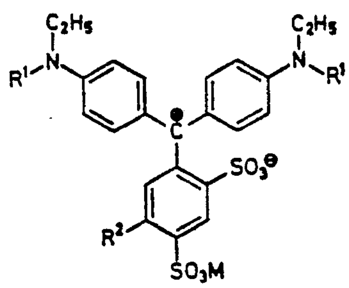

in der

$R^1$ eine niedere Alkyl- oder Aralkylgruppe
$R^2$ Wasserstoff oder eine Hydroxygruppe und
M ein Alkali- oder ein Erdalkaliäquivalent

bedeuten.

Unter einer niederen Alkylgruppe des Substituenten $R^1$ wird eine geradkettige oder verzweigte, gesättigte oder ungesättigte Kohlenwasserstoffkette mit 1 bis 6, vorzugsweise 1 bis 4 Kohlenstoffatomen verstanden. Der Aralkylrest des Substituenten $R^1$ weist eine Kohlenwasserstoffkette mit 1 bis 3 Kohlenstoffatomen auf, die mit einem oder mehreren aromatischen Resten, vorzugsweise Phenylrest, substituiert ist. Besonders bevorzugt ist der Benzylrest. Als Alkaliatome kommen vorzugsweise Natrium und Kalium, als Erdalkaliatome Magnesium und Calcium in Frage. Besonders geeignet sind die Farbstoffe Disulfinblau ($R^1$ = $C_2H_5$; $R^2$ = H; M = Na) sowie Patentblau AF (Acid Blue 7; $R^1$ = $CH_2$-$C_6H_5$; $R^2$ = H; M = Na).

Als Trägermaterialien sind alle üblichen Stoffe einsetzbar, die zur Imprägnierung mit immunologischen Substanzen geeignet sind. Im Sinne der vorliegenden Erfindung haben sich besonders bewährt: Cellulose, Papier oder auch Kunststoffmaterialien und Gemische hiervon.

Zur Herstellung eines Trägermaterials, das sowohl mit detektierbarer Substanz als auch mit Konjugat imprägniert ist, wird zweckmäßigerweise die detektierbare Substanz der Konjugattränklösung zugesetzt. Das Trägermaterial wird mit dieser Lösung getränkt und anschließend getrocknet. Ein weiterer Gegenstand der Erfindung ist demnach ein Mittel zur immunologischen Analytbestimmung in einer Probe enthaltend ein

Trägermaterial, welches mit einem markierten analytanalogen bzw. analytspezifischen Partner einer immunologischen Reaktion imprägniert ist, dadurch gekennzeichnet, daß es zusätzlich mit einer detektierbaren Substanz imprägniert ist.

Wird ein so hergestelltes Trägermaterial in einem heterogenen immunologischen Test eingesetzt, so wird sowohl der markierte analytanaloge bzw. analytspezifische Partner einer immunologischen Reaktion als auch die detektierbare Substanz aus dem Trägermaterial eluiert. Die detektierbare Substanz unterliegt sowohl auf dem Trägermaterial als auch in der Elutionslösung denselben Konzentrationsschwankungen wie der markierte Partner der immunologischen Reaktion. Die detektierbare Substanz beteiligt sich nicht an der immunologischen Reaktion. Nach Inkubation und Trennen des gebundenen und freien Anteils wird einmal die Markierung in üblicher Weise bestimmt und zum anderen die gelöste Menge der detektierbaren Substanz ermittelt. Aus von Probe zu Probe festgestellten Schwankungen des Meßsignals für die detektierbare Substanz kann unmittelbar auf analoge Konzentrationsschwankungen des markierten Partners der immunologischen Reaktion geschlossen werden.

Hierzu wird die Intensität des Meßsignals für die detektierbare Substanz, z. B. des Patentblau-Signals bei 650 nm, in mehreren Tests einer Testcharge gemessen, der Mittelwert gebildet und die Abweichung des jeweiligen Meßwertes vom Mittelwert in % berechnet. Dieser Prozentwert wird dann zur Korrektur des Meßwertes einer Analytbestimmung herangezogen.

Bei $\beta$-Galactosidase als Markierungsenzym wurde z. B. ein linearer Zusammenhang zwischen der Farbintensität der von dem Trägermaterial eluierten Farbstoffe, z. B. Disulfinblau oder anderen Patentblaufarbstoffen, und der eluierten Enzymmarkierung gefunden. Eine Änderung in der eluierten Enzymmarkierung führt zu einer entsprechenden Änderung des Meßwerts für die detektierbare Substanz, d. h. eine Schwankung in der Konjugatdosierung schlägt vollständig auf das Kontrollsignal durch, wobei hierunter im speziellen Fall eines Farbstoffs als detektierbare Substanz die Farbintensität verstanden wird.

Wird eine Probe mit dem zu bestimmenden Analyten mit einem Überschuß an analytspezifischem Partner der immunologischen Reaktion, der beispielsweise mit $\beta$-Galactosidase markiert ist und zusammen mit einer detektierbaren Substanz, z. B. Disulfinblau, aus einem Trägermaterial eluiert worden ist, inkubiert und wird anschließend der überschüssige Partner der immunologischen Reaktion mit an eine Matrix gebundenem Analyten abgefangen und schließlich eine charakteristische Meßgröße für die Markierung und für die detektierbare Substanz ermittelt, so erhält man als Meßergebnis:

1. Signal für detektierbare Substanz: $X_F$
2. Signal für Markierung: $X_A$.

Aus $X_A$ läßt sich aus einer in üblicher Weise gewonnenen Eichkurve die Konzentration des Analyten $C_A$ ermitteln.

Diese Analytkonzentration kann anhand des Signals für die detektierbare Substanz nach folgender allgemeiner Beziehung korrigiert werden:

$$C_{korrigiert} = C_A + f \cdot \frac{(\overline{X}_F - X_F)}{\overline{X}_F} \cdot C_A$$

Hierin bedeuten:

| | | |
|---|---|---|
| $C_{korrigiert}$ | = | korrigierte Analytkonzentration (verbessertes Meßergebnis) |
| $C_A$ | = | Analytkonzentration (aus Meßwert über Eichkurve ermittelt) |
| $X_F$ | = | Signal für die detektierbare Substanz der jeweiligen Bestimmung |
| $\overline{X}_F$ | = | Mittelwert Signal für die detektierbare Substanz für eine Testcharge |
| f | = | analytspezifischer empirischer Faktor |

Den Mittelwert $\overline{X}_F$ erhält man, indem man die Signale für die detektierbare Substanz von mehreren Tests einer bestimmten Testcharge in oben erwähnter Weise ermittelt und hieraus den Mittelwert errechnet. Unter einer Testcharge werden hierbei alle Tests verstanden, bei deren Herstellung zur Imprägnierung des Trägermaterials dieselbe Tränklösung mit dem markierten Partner der immunologischen Reaktion und der detektierbaren Substanz verwendet worden ist.

Der analytspezifische Faktor f wird ermittelt, indem man in vorstehender Weise mehrere Proben mit bekannten Analyt-Konzentrationen vermißt, die gefundenen Meßdaten in obige Gleichung einsetzt und unter Berücksichtigung des chargenspezifischen Mittelwertes $\overline{X}_F$ den jeweils zugehörigen Faktor f als Mittelwert berechnet. Als Konzentration $C_{korrigiert}$ ist hierbei jeweils die vorbekannte Analytkonzentration einzusetzen.

Es hat sich gezeigt, daß auf diese Weise ein für einen bestimmten Analyten spezifischer Faktor erhalten wird.

Dieser beträgt beispielsweise für Digoxin 0,5 und für T3 0,65. Mit Hilfe des erfindungsgemäßen Verfahrens ist es möglich, die Stabilität von Konjugattränklösungen durch Messung nach verschiedenen Zeiten zu kontrollieren, Bilanzierungsschwierigkeiten bei der Überprüfung von Trocknungsverlusten und Wiederfindung zu vermeiden, Chargenhomogenitäten zu überprüfen, Schwankungen von Markierungseigenschaften, wie z. B. der enzymatischen Aktivität, die durch Rohstoffeinflüsse bedingt sein können, durch Referenzmessung zu erfassen und den Einfluß von Konzentrationsschwankungen des Konjugats auf den gesamten immunologischen Test zu überprüfen.

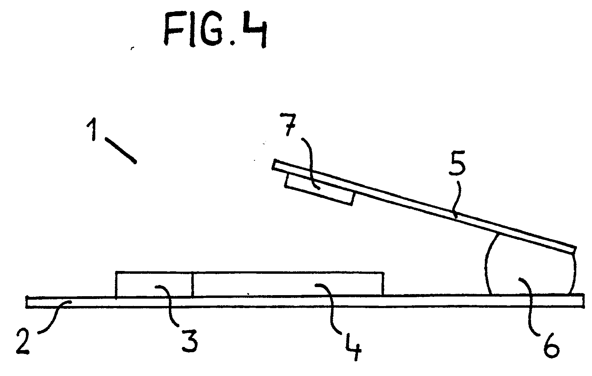

Ein Beispiel für eine Vorrichtung, mit der das erfindungsgemäße Verfahren durchgeführt werden kann, zeigt Fig. 4.

Die Vorrichtung 1 besteht aus einer Grundfolie 2, auf welcher das mit einer detektierbaren Substanz standardisierte erfindungsgemäße Konjugatvlies 3, einer Matrix 4, die einen Überschuß des zu bestimmenden Analyten in trägergebundener Form enthält, sowie eine Folie 5, die über eine Klebestelle 6 an der Grundfolie 2 befestigt ist. Auf die Folie 5 ist ein Substratfilm 7 auf der der Matrix zugewandten Seite der Folie aufgetragen.

Zur Bestimmung eines Analyten in einer Flüssigkeit wird die Probe auf das Konjugatvlies 3 aufgegeben. Die Flüssigkeit mit den aus Vlies 3 gelösten Stoffen wird durch Kapillarkräfte in die Matrix 4 transportiert. Zur Messung der Menge an Markierung wird die Folie 5 mit dem Substratfilm 7 auf die Matrix aufgepreßt. Nach einem festgelegten Zeitpunkt wird der Meßwert für die detektierbare Substanz und der durch die Reaktion der Markierung mit dem Substrat verursachte Meßwert ermittelt. Die Analytkonzentration wird, wie oben beschrieben, korrigiert.

Die vorliegende Erfindung ist selbstverständlich nicht auf eine Vorrichtung gemäß Fig. 4 beschränkt. Für heterogene Immunoassays einsetzbare Vorrichtungen können ohne weiteres von einem Fachmann in erfindungsgemäßer Weise modifiziert werden.

Ein weiterer Gegenstand der Erfindung ist die Verwendung eines Trägermaterials welches mit einem aufgrund seiner physikalischen Eigenschaften detektierbaren Stoffes zur Kontrolle der Konzentration eines von einem Trägermaterial eluierten markierten Partners einer immunologischen Reaktion imprägniert ist. Solche Stoffe, die aufgrund ihrer physikalischen Eigenschaften detektierbar sind, sind insbesondere fluoreszierende Verbindungen oder Farbstoffe. Besonders bevorzugt sind zur Kontrolle der Konzentration ß-Galactosidasemarkierter Antikörper Patentblaufarbstoffe, wie Disulfinblau.

In Fig. 1 ist ein Einsatzelement zur Bestimmung eines Analyten gezeigt, welches im erfindungsgemäßen Verfahren eingesetzt werden kann. Die Bezugszeichen haben dabei folgende Bedeutung:

| | |
|---|---|
| a | Kammer, welche ein mit Hilfsstoffen imprägniertes Vlies enthält. |
| b | Kammer, welche ein Vlies enthält, das mit Anti-T3-MAK-Fab-Fragment / ß-Galaktosidase-Konjugat imprägniert ist. |
| c | Kammer mit Trennmatrix, die T3 unlöslich gebunden enthält. |
| AK | Kammer mit einem Leervlies. |
| d | Kammer, welche ein mit Chlorphenolrotgalactosid imprägniertes Vlies enthält. |
| VK 1-3 | Ventilkammern |
| P | Probenauftragskammer |
| K | Küvette |
| Fig. 2 | zeigt eine Eichkurve zur Bestimmung von T3. |
| Fig. 3 | zeigt eine Eichkurve zur Bestimmung von Digoxin. |
| Fig. 4 | zeigt eine oben erläuterte Vorrichtung zur Durchführung des erfindungsgemäßen Verfahrens. |

Das erfindungsgemäße Verfahren zur Bestimmung eines Partners einer immunologischen Reaktion und die Verwendung hierzu geeigneter detektierbarer Substanzen wird durch die folgenden Beispiele näher erläutert.

Beispiel 1

Herstellung eines mit Patentblaufarbstoff standardisierten Konjugatvlieses

Vliesmaterial (80 % Polyester, 20 % Zellwolle, mit Kuralon® verfestigt; Saugkapazität ca. 600 ml/m$^2$ ) wird mit einer Lösung der folgenden Zusammensetzung getränkt und mit Umluft getrocknet.

Tränklösung
Natrium-HEPES-Puffer, 100 mM, pH 7.25
Polyoxygelatine 1 %

Magnesium-Aspartat 5 mM
Anti-T3-monoklonaler Antikörper Fab-Fragment 280 U/1
Disulfinblau 0.015 ‰

Beispiel 2

Bestimmung von T3

A) Bestimmung von T3 als Beispiel eines Haptens gemäß EP-A 0 167 171 unter Verwendung eines Einsatzelementes gemäß Fig. 1 (Konjugatvlies ohne detektierbare Substanz)

Bestückung des Einsatzelementes gemäß Fig. 1

| | |
|---|---|
| Kammer a) | 2 Vliese, je 0,5 mm dick<br>imprägniert mit<br>Na-HEPES, 125 mM, pH 7,25<br>Tween®20, 0,25 %<br>bzw.<br>ANS 0,06 % (ANS = Anilino Naphthalin Sulfonsäure),<br>Tween®20, 0,01 % |
| Kammer b) | 2 Leervliese, 1 Konjugatvlies, jeweils 0,5 mm dick, imprägniert mit<br>Anti-T3-monoklonaler Antikörper Fab-Fragment, gebunden an $\beta$-Galactosidase 1,6 mU (Fab-E)<br>Na-HEPES, 100 mM, pH 7,25<br>Polyoxygelatine 1 %<br>Mg-Aspartat, 5 mM |
| Kammer c) | Trenn-Matrix, 2 Vliese, je 0,7 mm dick T3, unlöslich gebunden an das Matrixvlies |
| Kammer AK) | 1 Vlies von 1 mm Dicke |
| Kammer d) | 1 Vlies von 0,5 mm Dicke, imprägniert mit<br>Na-HEPES, 100 mM, pH 7,25<br>Borsäure, 5 mM<br>Chlorphenolrotgalactosid 18 mM |

Flüssigkeitspipettierungen:

Es werden 5 µl Probelösung in die Probenauftragskammer P pipettiert, gefolgt von 50 µl Diluens (physiologische Kochsalzlösung). Die Mischung der Komponenten erfolgt durch den Pipettiervorgang. In Kammer c werden 40 µl Diluens pipettiert.

Reaktionsführung:

| | |
|---|---|
| 1. Zentrifugation | Die auf die Matrix (c) pipettierte Flüssigkeit wird in die Auffangkammer (AK) geschleudert. Durch diesen Waschvorgang der Matrix werden Haptenmoleküle, deren Bindung zur Matrix sich während der Lagerung gelöst hatte, entfernt. Diese Moleküle würden ansonsten wie Probe wirken und das Ergebnis verfälschen. Die Probeflüssigkeit überströmt gleichzeitig Kammer a, löst dabei die dort befindlichen Reagenzien und es kann in der Ventilkammer VK1 eine Vorreaktion stattfinden. Die Ablösereaktion, bei der ANS das T3 aus der Bindung mit den Bindungsproteinen (vorwiegend Thyroxin bindendes Globulin (TBG)) löst, muß nicht vollständig sein, da eine Weiterreaktion in VK2 hier möglich ist. |
| 1. Stillstand | Die Probe geht in Kammer b, hier wird das Fab-Enzym-Konjugat abgelöst. |
| 2. Zentrifugation | Die Probe wird in VK2 befördert. Diese Zentrifugation wird für fünf Minuten aufrechterhalten. Dabei reagiert das T3 aus der Probe mit dem Fab-Enzym-Konjugat (Fab-E) zu dem Komplex T3•Fab-E. |
| 2. Stillstand | Die Probe geht auf Vlies in Kammer c. Hier bindet überschüssiges Fab-E an die Matrix über das matrixgebundene T3. Diese Reaktion dauert fünf Minuten. |
| 3. Zentrifugation | Der erste Teil der Flüssigkeit füllt die Auffangkammer vollständig, der größere Teil wird über Vlies in Kammer d in die VK 3 geschleudert. Von d wird dabei das Substrat heruntergelöst. |
| 3. Stillstand | Die reagierende Lösung verläßt VK 3. |
| Meßzentrifugation | Die Lösung wird in die Küvette transportiert und die Reaktion absorptionsphotometrisch bei 578 nm verfolgt. Die Konjugatmoleküle, welche T3 aus der Probe |

gebunden hatten, konnten die Matrix passieren und es befindet sich jetzt eine der Konzentration T3 entsprechende Menge Enzym in der Küvette. Die gemessene Farbzunahme pro Zeiteinheit ist daher ein Maß für die Konzentration T3 in der Probe.

Wegen der Proportionalität zwischen Enzym und Analyt werden lineare Eichkurven erhalten. Dies zeigt Fig. 2.

B) Bestimmung von T3, wie vorstehend unter A beschrieben, unter Verwendung eines Einsatzelementes gemäß Fig. 1, wobei das Konjugatvlies in Kammer b zusätzlich mit Disulfinblau, wie in Beispiel 1 beschrieben, imprägniert ist.

Die Bestückung des Einsatzelementes gemäß Fig. 1 erfolgt in analoger Weise wie unter A beschrieben. Lediglich in Kammer b wird anstelle des unter A verwendeten Konjugatvlieses ein nach Beispiel 1 hergestelltes Konjugatvlies mit Disulfinblau als detektierbare Substanz verwendet.

Die Abfolge der Zentrifugationen und Stillstände erfolgt wie in Abschnitt A unter Reaktionsführung beschrieben. Zusätzlich zu der bei 578 nm gemessenen Absorptionsänderung, die durch die enzymatische Reaktion hervorgerufen wird, wird bei 650 nm die Absorption des Disulfinblaus bestimmt.

C) Vergleich der Ergebnisse nach A) und B)

Zum Vergleich der Ergebnisse nach Methode A) und Methode B) werden am geeignetsten die Variationskoeffizienten herangezogen.

Hierzu werden jeweils n Bestimmungen mit Standardlösungen a, b und c mit bekannten T3-Konzentrationen (1.74 ng/ml, 2.82 ng/ml bzw. 5.17 ng/ml) nach A) bzw. B) durchgeführt. Aus den Meßwerten $X_A$ für das Enzymsubstrat werden mittels der Eichkurve (Fig. 2) die zugehörigen T3-Konzentrationen $C_A$ ermittelt. Bei Methode A) erhält man hieraus direkt nach bekannten mathematischen Methoden den Variationskoeffizienten VK(A). Bei Methode B) wird zusätzlich zu dem Substratsignal ein Signal für das Disulfinblau $X_F$ gemessen. Aus den Einzelsignalen $X_F$ wird der für die Testcharge gültige Mittelwert des Disulfinblaus $\overline{X}_F$ berechnet. Über die oben genannte Beziehung werden zu den jeweiligen Meßwerten $X_F$ und $C_A$ die zugehörigen korrigierten T3-Konzentrationen $C_{korrigiert}$ ermittelt, wobei als analytspezifischer empirischer Faktor f der oben angegebene Wert von 0,65 eingesetzt wird. Mit diesen läßt sich der Variationskoeffizient VK(B) berechnen. Die Ergebnisse sind in folgender Übersicht zusammengestellt:

| Standard lösung | T3-Konzentration ng/ml | n | VK (A) % | VK (B) % |
|---|---|---|---|---|
| a | 1.74 | 32 | 6.5 | 5.1 |
| b | 2.82 | 32 | 5.6 | 4.4 |
| c | 5.17 | 32 | 4.5 | 3.4 |

Die vorstehenden Daten zeigen, daß die Variationskoeffizienten für die erfindungsgemäße Methode B deutlich kleiner sind als für die Methode A gemäß dem Stand der Technik.

## Beispiel 3

### Bestimmung von Digoxin

A) Bestimmung ohne Zusatz einer detektierbaren Substanz

Das Verfahren ist analog dem Beispiel 2 A), jedoch werden für das Konjugat Fab-Fragmente eines Antikörpers gegen Digoxin verwendet. Die Matrix besteht in diesem Fall aus an die Festphase gebundenem Digoxin. Eine so erhaltene Eichkurve zeigt Fig. 3.

B) Bestimmung von Digoxin mit Zusatz einer detektierbaren Substanz

Es wird wie unter 2 A) beschrieben vorgegangen unter Verwendung eines Einsatzelementes gemäß Fig. 1. Das Konjugatvlies in Kammer b ist zusätzlich mit Disulfinblau, wie in Beispiel 1 beschrieben, imprägniert.

C) Vergleich der Ergebnisse nach A) und B)

Zum Vergleich der Ergebnisse nach Methode A) und Methode B) werden am geeignetsten die Variationskoeffizienten herangezogen.

Hierzu werden jeweils n Bestimmungen mit Standardlösungen a, b und c mit bekannten Digoxin-Konzentrationen (2.03 ng/ml, 4.02 ng/ml bzw. 6.43 ng/ml) nach A) bzw. B) durchgeführt. Aus den Meßwerten $X_A$ für das Enzymsubstrat werden mittels der Eichkurve (Fig. 3) die zugehörigen Digoxin-Konzentrationen $C_A$ ermittelt. Bei Methode A) erhält man hieraus direkt nach bekannten mathematischen Methoden den Variationskoeffizienten VK(A). Bei Methode B) wird zusätzlich zu dem Substratsignal ein Signal $X_F$ für das Disulfinblau gemessen. Aus den Einzelsignalen $X_F$ wird der für die Testcharge gültige Mittelwert $\overline{X}_F$ des Disulfinblaus berechnet. Über die oben genannte Beziehung werden zu den jeweiligen Meßwerten $X_F$ und $C_A$ die zugehörigen korrigierten Digoxin-Konzentrationen $C_{korrigiert}$ ermittelt, wobei als analytspezifischer empirischer Faktor f der oben angegebene Wert von 0,5 eingesetzt wird.

Mit diesen läßt sich der Variationskoeffizient VK(B) berechnen. Die Ergebnisse sind in folgender Übersicht zusammengestellt:

| Standard lösung | Digoxin-Konzentration ng/ml | n | VK (A) % | VK (B) % |
|---|---|---|---|---|
| a | 2.03 | 51 | 3.3 | 2.8 |
| b | 4.02 | 32 | 7.7 | 5.4 |
| c | 6.43 | 32 | 4.1 | 2.8 |

Die vorstehenden Daten zeigen, daß die Variationskoeffizienten für die erfindungsgemäße Methode B deutlich kleiner sind als für die Methode A gemäß dem Stand der Technik.

**Patentansprüche**

1. Heterogenes immunologisches Verfahren zur Analytbestimmung in einer Probe, bei dem mindestens ein Partner der immunologischen Reaktion von einem Trägermaterial gelöst wird, wobei die analytenthaltende Probe mit einem von einem Trägermaterial gelösten, markierten analytspezifischen Partner einer immunologischen Reaktion inkubiert, mit einem Überschuß des zu bestimmenden Analyten in einer gebundenen Form kontaktiert, der gebundene und der freie Anteil des so entstandenen Immunkomplexes getrennt und die Markierung im freien oder gebundenen Anteil gemessen wird, dadurch gekennzeichnet, daß das Trägermaterial zusätzlich eine detektierbare, die immunologische Reaktion nicht beeinflussende Substanz enthält, die zusammen mit dem Partner der immunologischen Reaktion eluiert wird und man einen für die Konzentration der detektierbaren Substanz in der Lösung enthaltenen Meßwert mit dem Meßwert für die Analytkonzentration in der Probe korreliert.

2. Verfahren gemäß Anspruch 1, dadurch gekennzeichnet, daß die detektierbare Substanz anhand ihrer physikalischen Eigenschaften detektierbar ist.

3. Verfahren gemäß Anspruch 1 oder 2, dadurch gekennzeichnet, daß als detektierbare Substanz eine fluoreszierende Verbindung oder ein Farbstoff eingesetzt wird.

4. Verfahren gemäß Anspruch 3, dadurch gekennzeichnet, daß als Farbstoff ein Patentblaufarbstoff eingesetzt wird.

5. Verwendung eines Trägermaterials, welches mit einem analytspezifischen Partner einer immunologischen Reaktion und einer detektierbaren, die immunologische Reaktion nicht beeinflussenden, zusammen mit dem Partner der immunologischen Reaktion eluierbaren, detektierbaren Subsatanz imprägniert ist, in heterogenen immunologischen Verfahren zur Analytbestimmung in einer Probe gemäß Anspruch 1.

6. Verwendung eines Trägermaterials gemäß Anspruch 5, dadurch gekennzeichnet, daß die Substanz eine fluoreszierende Verbindung oder Farbstoff ist.

7. Verwendung eines Trägermaterials gemäß Anspruch 6, dadurch gekennzeichnet, daß als Farbstoff ein Patentblaufarbstoff eingesetzt wird.

8. Mittel zur immunologischen Analytbestimmung in einer Probe, enthaltend ein Trägermaterial, welches mit einem markierten analytspezifischen Partner einer immunologischen Reaktion und einer die immunologische Reaktion nicht beeinflussenden, zusammen mit dem Partner der immunologischen Reaktion eluierbaren, detektierbaren Substanz imprägniert ist.

9. Mittel gemäß Anspruch 8, dadurch gekennzeichnet, daß die detektierbare Substanz eine fluoreszierende Verbindung oder ein Farbstoff ist.

10. Mittel gemäß Anspruch 9, dadurch gekennzeichnet, daß der Farbstoff ein Patentblaufarbstoff ist.

## Claims

1. Heterogeneous immunological process for the analyte determination in a sample in which at least one partner of the immunological reaction is dissolved from a carrier material, whereby the analyte-containing sample is incubated with a labelled, analyte-specific partner of an immunological reaction dissolved from a carrier material, contacted with an excess of the analyte to be determined in a bound form, the bound and the free part of the immune complexes so formed separated and the labelling measured in the free or bound part, characterised in that the carrier material additionally contains a detectable substance not influencing the immunological reaction which is eluted together with the partner of the immunological reaction and one correlates a measurement value obtained for the concentration of the detectable substance in the solution with the measurement value for the analyte concentration in the sample.

2. Process according to claim 1, characterised in that the detectable substance is detectable on the basis of its physical properties.

3. Process according to claim 1 or 2, characterised in that a fluorescing compound or a coloured material is used as detectable substance.

4. Process according to claim 3, characterised in that an acid blue is used as coloured material.

5. Use of a carrier material which is impregnated with an analyte-specific partner of an immunological reaction and a detectable substance not influencing the immunological reaction, elutable together with the partner of the immunological reaction, in heterogeneous immunological processes for the analyte determination in a sample according to claim 1.

6. Use of a carrier material according to claim 5, characterised in that the substance is a fluorescing compound or coloured material.

7. Use of a carrier material according to claim 6, characterised in that an acid blue is used as coloured material.

8. Agent for the immunological analyte determination in a sample, containing a carrier material which is impregnated with a labelled, analyte-specific partner of an immunological reaction and a detectable substance not influencing the immunological reaction elutable together with the partner of the immunological reaction.

9. Agent according to claim 8, characterised in that the detectable substance is a fluorescing compound or a coloured material.

10. Agent according to claim 9, characterised in that the coloured material is an acid blue.

## Revendications

1. Procédé immunologique hétérogène pour la détermination analytique d'un échantillon, dans lequel au

moins un partenaire de la réaction immunologique est dissous dans un matériau de support, procédé dans lequel l'échantillon contenant la substance à analyser est incubé avec un partenaire d'une réaction immunologique qui est marqué, dissous dans un matériau de support, et spécifique à la substance à analyser, est mis en contact sous une forme liée avec une quantité en excès de la substance à déterminer, la partie libre et la partie liée de l'immunocomplexe ainsi formé sont séparées et le marquage dans la partie libre ou liée est mesuré, caractérisé en ce que le matériau de support contient en outre une substance détectable, n'influençant pas la réaction immunologique, qui est éluée conjointement avec le partenaire de la réaction immunologique, et que l'on effectue la corrélation entre une grandeur de mesure obtenue pour la concentration de la substance à détecter dans la solution et la grandeur de mesure pour la concentration de la substance à analyser dans l'échantillon.

**2.** Procédé selon la revendication 1, caractérisé en ce que la substance détectable peut être détectée en se basant sur ses propriétés physiques.

**3.** Procédé selon la revendication 1 ou 2, caractérisé en ce que comme substance détectable, on met en oeuvre un composé fluorescent ou un colorant.

**4.** Procédé selon la revendication 3, caractérisé en ce que le colorant mis en oeuvre est un alphazurin.

**5.** Utilisation d'un matériau de support qui est imprégné avec un partenaire d'une réaction immunologique qui est spécifique à la substance à analyser et avec une substance détectable, n'influençant pas la réaction immunologique, éluable conjointement avec le partenaire de la réaction immunologique, dans un procédé immunologique hétérogène pour la détermination d'une substance à analyser dans un échantillon, selon la revendication 1.

**6.** Utilisation d'un matériau de support selon la revendication 5, caractérisée en ce que la substance est un composé flucrescent ou un colorant.

**7.** Utilisation d'un matériau de support selon la revendication 6, caractérisée en ce que comme colorant on utilise un alphazurin.

**8.** Agent pour la détermination immunologique d'une substance à analyser dans un échantillon, contenant un matériau de support qui est imprégné avec un partenaire d'une réaction immunologique qui est marqué et spécifique à la substance à analyser, et avec une substance détectable, n'influençant pas la réaction immunologique, éluable avec le partenaire de la réaction immunologique.

**9.** Agent selon la revendication 8, caractérisé en ce que la substance détectable est un composé fluorescent ou un colorant.

**10.** Agent selon la revendication 9, caractérisé en ce que le colorant est un alphazurin.

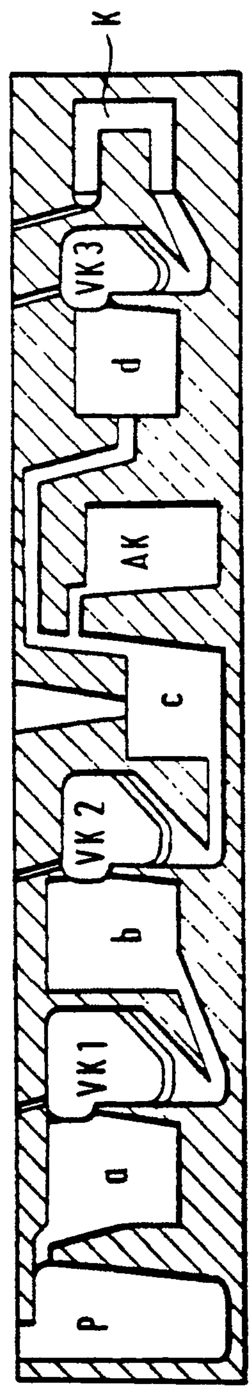


FIG. 1

FIG. 2

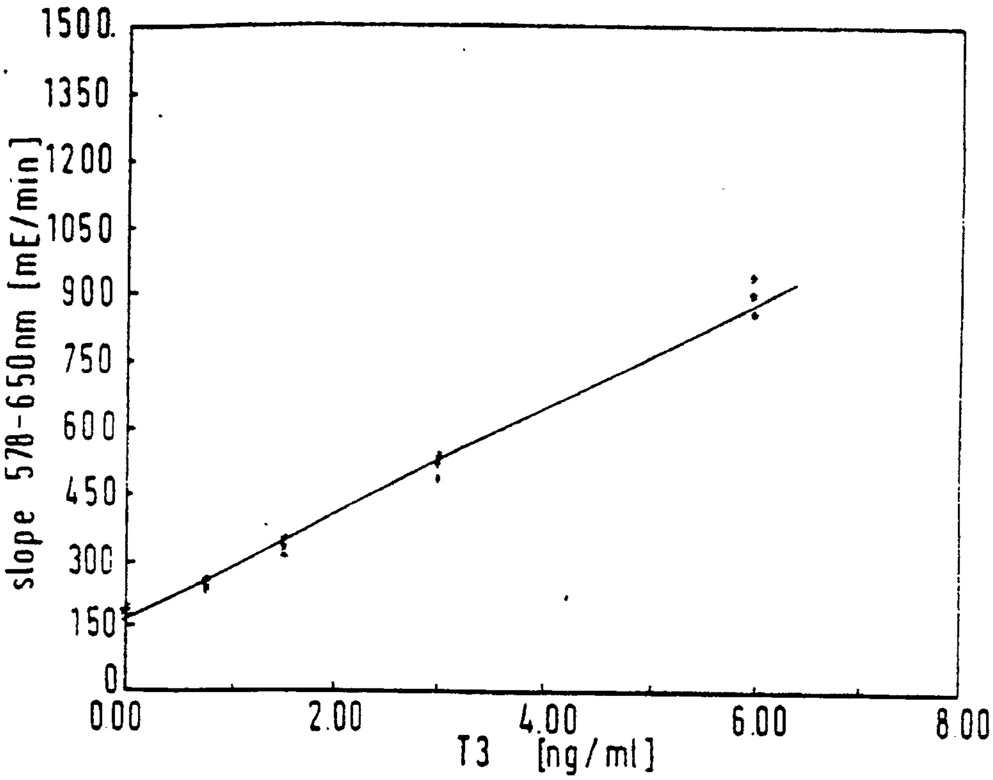
1500
1350
1200
1050
900
750
600
450
300
150
0
slope 578-650nm [mE/min]
0.00
2.00
4.00
6.00
8.00
T3 [ng/ml]

FIG. 3

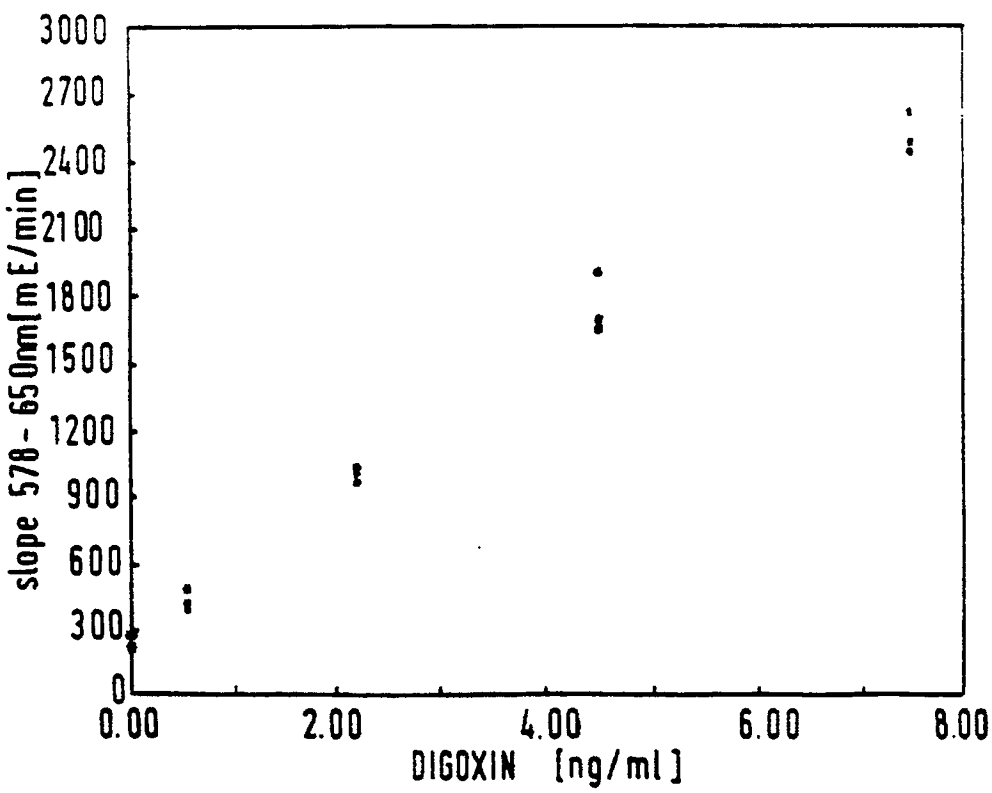
3000
2700
2400
2100
1800
1500
1200
900
600
300
0
slope 578-650nm[mE/min]
0.00
2.00
4.00
6.00
8.00
DIGOXIN [ng/ml]

FIG. 4